# EUROPEAN PATENT APPLICATION

(11) **EP 3 973 857 A1**
(43) Date of publication of application: **30.03.2022**
(21) Application number: 20198847.4
(22) Date of filing: 29.09.2020
(51) Int. Cl.: A61B 5/021, A61B 5/11, A61B 8/08, A61B 8/04

(54) **ESTIMATING CENTRAL BLOOD PRESSURE**

(71) Applicant: Koninklijke Philips N.V., 5656 AG Eindhoven (NL)
(72) Inventor: DE WILD, Nico Maris Adriaan, 5656 AE Eindhoven (NL); LAU, Kevin Daniel Seng Hung, 5656 AE Eindhoven (NL); BARAGONA, Marco, 5656 AE Eindhoven (NL); MAESSEN, Ralph Theodorus Hubertus, 5656 AE Eindhoven (NL); BINGLEY, Peter, 5656 AE Eindhoven (NL)
(74) Representative: Philips Intellectual Property & Standards

(57) **Abstract**

A device is for estimating the central blood pressure of a subject. The device comprises a sensor patch comprising an array of sensors configured to measure an indication of the blood pressure of a peripheral blood vessel and perform ultrasound imaging of the peripheral blood vessel. The device also comprises a processor configured to obtain a peripheral pressure signal comprising a pressure waveform from the indication of the blood pressure from the sensor patch and derive an image of the peripheral blood vessel from the signals received from the sensor patch. A vessel diameter is determined from the image of the peripheral blood vessel over time comprising a vessel diameter waveform and an estimate of the central blood pressure of the subject is derived from the pressure waveform and/or the vessel diameter waveform.

## Description

### FIELD OF THE INVENTION

The invention relates to the estimation of the central blood pressure of a subj ect.

### BACKGROUND OF THE INVENTION

The measurement of blood pressure is routinely utilized in clinical practice to assess and categorize the general health of patients. Prolonged values of increased blood pressure (hypertension) are associated with multiple complications, e.g. heart failure, stroke, chronic kidney disease, etc.

The measurement of the maximum (systolic) pressure varies in dependence upon the location in the arterial tree, as amplification effects arise from pressure wave reflections from vessel bifurcations and variations in vessel caliber. Thus, the systolic pressure in a peripheral location (e.g. the brachial artery) is not equal to the systolic pressure in a central location such as the aorta.

The central aortic pressure provides important diagnostic information, e.g. risk factors for adverse outcomes such as mortality, and efficacy of treatments, such as antihypertensive treatments, have different effects upon central and peripheral (brachial) pressures. Thus, a non-invasive estimation of the central pressure waveform would be a key indicator.

The most popular techniques for central pressure estimation rely upon the measurement of peripheral pressure. These techniques can be divided into two approaches: a force-based measurement of vessel displacement (e.g. applanation tonometry) or an ultrasound-based imaging of vessel diameter.

In the force-based approach, the variation in vessel diameter arising from the blood pressure waveform is measured via an external force measurement at a peripheral location (e.g. the arm or neck). This measurement is then calibrated to produce a surrogate peripheral blood pressure waveform. This waveform is subsequently transformed to a central waveform using a transfer function in the frequency domain. This approach is utilized by Sphymocor (trade mark) devices. Other devices also utilize the same force-based approach but do not mathematically transform the pressure signal.

The application of force-based measurements (applanation tonometry) has been implemented to the carotid artery to derive central pressure waveforms. However, the depth of the carotid vessel requires a skilled operator to obtain a reliable signal by such force-based techniques.

In the ultrasound-based approach, the variation in vessel diameter arising from the blood pressure waveform is measured via an ultrasound image. The waveform is then calibrated to produce a surrogate blood pressure waveform.

The use of ultrasound is advantageous as the above-described problems are avoided. Academic studies report the use of ultrasound imaging to determine a surrogate pressure signal.

The different methods produce different quality measurements in different situations. It would be desirable to have an improved method of estimating a central blood pressure, which can produce good quality results in a broad range of application situations.

### SUMMARY OF THE INVENTION

The invention is defined by the claims.

According to examples in accordance with an aspect of the invention, there is provided a device for estimating the central blood pressure of a subject, the device comprising:
a sensor patch comprising an array of sensors configured to:
   measure an indication of the blood pressure of a peripheral blood vessel; and
   perform ultrasound imaging on the peripheral blood vessel, wherein the same sensors are used for blood pressure measurement and ultrasound imaging; and
a processor configured to:
   obtain a peripheral pressure signal comprising a pressure waveform from the indication of the blood pressure from the sensor patch;
   derive an image of the peripheral blood vessel from the signals received from the sensor patch;
   determine a vessel diameter from the image of the peripheral blood vessel over time as a vessel diameter waveform; and
   derive an estimate of the central blood pressure waveform of the subject from the pressure waveform and/or the vessel diameter waveform.

The sensor patch has the capability of measuring an indication of blood pressure at a peripheral location and to perform ultrasound imaging of the same location. The indication of blood pressure could be, for example, the force at the skin of the subject caused by changes in blood pressure at the peripheral location. The changes in blood vessel diameter can be measured from imaging the blood vessel with the ultrasound imaging capability. Both of the peripheral pressure signal and the vessel diameter can be used to estimate the central blood pressure from a peripheral blood vessel in a non-invasive way. By combining both capabilities into a single sensor patch, a more robust and reliable estimation can be made of the central blood pressure.

The processor may be configured to determine the vessel diameter by applying an automatic tracking algorithm to the image of the peripheral blood vessel in color Doppler mode.

Applying an automatic tracking algorithm to the peripheral blood vessel image (in color Doppler mode) allows a reliable and consistent measurement of the diameter of the peripheral blood vessel with respect to time.

The array of sensors may comprise:
capacitive micro-machined ultrasound transducers (cMUTs);
piezoelectric sensors; or
piezoresistive sensors.

Thus, various sensors may be configurable to function both as ultrasound imaging sensors and pressure sensors.

The processor may be further adapted to:
determine a quality factor for each of a plurality of peripheral pressure waveform cycles and vessel diameter waveform cycles;
create an ensemble pressure waveform based on the pressure waveform cycles and their respective quality factors; and
create an ensemble diameter waveform based on the vessel diameter waveform cycles and their respective quality factors,
wherein estimating the central blood pressure is based on the ensemble pressure waveform and/or the ensemble diameter waveform.

The ensemble waveforms can be determined by, for example, using different weightings on the peripheral pressure waveform cycles and the vessel diameter waveform cycles based on the quality, the regularity and/or comparing the signals to expected signals (e.g. using a lower weighting for low quality/unexpected signals).

For example, the vessel diameter may not be determined perpendicularly to the vessel. In this case, the quality factor of the vessel diameter is low as it is not an accurate representation of the real vessel diameter. Thus, the peripheral pressure waveforms may be solely used to estimate the central blood pressure.

The processor may be adapted to derive an estimate of the central blood pressure by:
applying a frequency-based transfer function to the ensemble pressure waveform and/or the ensemble diameter waveform; or
using a physics-based model to transform the ensemble pressure waveform and/or the ensemble diameter waveform to an estimate of the central blood pressure.

The processor may be further adapted to:
calibrate a mean value and a diastolic value of the estimated central blood pressure to a cuff mean value and a cuff diastolic value obtained from pressure cuff measurements.

The mean blood pressure and diastolic blood pressure value can generally be assumed as constant in different parts of the body. However, the maximum value of the waveform varies based on the anatomical location. Thus, once a waveform is obtained, the shape of the waveform can be calibrated to accurate blood pressure values by using real mean and diastolic values from pressure cuff measurements.

The device may further comprise:
a housing element for housing the sensor patch and the processor, wherein the housing element comprises an opening and wherein the opening is for a sensing area of the sensor patch;
a strap attached the housing element; and
a skin interface at the opening of the housing element.

The invention further provides a method for estimating central blood pressure, the method comprising:
measuring an indication of blood pressure of a peripheral blood vessel to derive a pressure waveform from sensors of a sensor patch;
performing ultrasound imaging on the peripheral blood vessel using the same sensors as are used for the indication of blood pressure measurement;
determining a vessel diameter from the image of the peripheral blood vessel over time as a vessel diameter waveform; and
deriving an estimate of the central blood pressure waveform of the subject from the pressure waveform and/or the vessel diameter waveform.

Determining the vessel diameter may be based on applying an automatic tracking algorithm to the image in color Doppler mode.

The method may further comprise:
The method may further comprise:
determining a quality factor for a plurality of peripheral pressure waveform cycles and a plurality of vessel diameter waveform cycles;
creating an ensemble pressure waveform based on the pressure waveform cycles and their respective quality factors; and
creating an ensemble diameter waveform based on the vessel diameter waveform cycles and their respective quality factors,
wherein estimating the central blood pressure is based on the ensemble pressure waveform and/or the ensemble diameter waveform.

Estimating the central blood pressure may be based on:
applying a frequency-based transfer function to the ensemble pressure waveform and/or the ensemble diameter waveform; or
using a physics-based model to transform the ensemble pressure waveform and/or the ensemble diameter waveform to an estimate of the central blood pressure.

The method may further comprise calibrating a mean value and a diastolic value of the estimated central blood pressure to a cuff mean value and a cuff diastolic value obtained from pressure cuff measurements.

The invention also provides a computer program comprising computer program code which, when executed by a processor, causes the processor to perform the methods as described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a representation of sensor patch for measuring a peripheral blood vessel diameter and blood pressure;
Fig. 2 shows a pressure waveform measured at the skin of a subject;
Fig. 3 shows the measured diameter between the opposing inner surfaces and between the opposing outer surfaces of a blood vessel, based on image analysis;
Fig. 4 shows a graph with a velocity waveform (for three cycles) and a corresponding vessel diameter waveform;
Fig. 5 shows example pressure waveforms at different anatomical locations;
Fig. 6 shows an example of a capacitive cMUT sensor;
Fig. 7 shows a known design of cMUT element (a so-called cell) for use in the sensor patch 102;
Figs. 8 and 9 depict operating principles of such a cMUT element;
Fig. 10 shows one way to measure pressure and perform imaging the same sensors of a sensing patch; and
Fig. 11 shows a wearable device with a sensing patch.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figs. are merely schematic and are not drawn to scale. It should also be understood that the same reference numerals are used throughout the Figs. to indicate the same or similar parts.

The invention provides a device for estimating the central blood pressure of a subject. The device comprises a sensor patch comprising an array of sensors configured to measure an indication of the blood pressure of a peripheral blood vessel and perform ultrasound imaging of the peripheral blood vessel. The device also comprises a processor configured to obtain a peripheral pressure signal comprising a pressure waveform from the indication of the blood pressure from the sensor patch and derive an image of the peripheral blood vessel from the signals received from the sensor patch. A vessel diameter is determined from the image of the peripheral blood vessel over time comprising a vessel diameter waveform and an estimate of the central blood pressure of the subject is derived from the pressure waveform and/or the vessel diameter waveform.

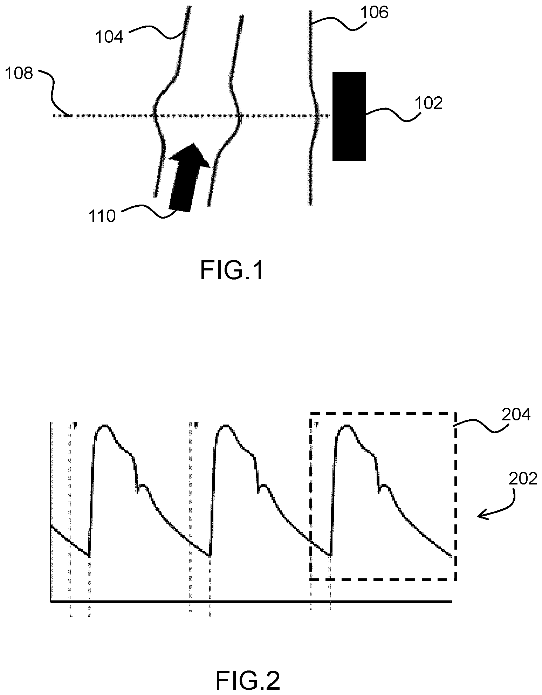

Fig. 1 shows a representation of sensor patch 102 for measuring a peripheral blood vessel diameter and blood pressure. The ultrasound patch 102 has an array of sensors for ultrasound imaging, from which the diameter of a peripheral blood vessel 104 can obtained, and also for measuring the force at the skin 106 which is representative of the blood pressure. The sensor patch 102 measures both of these at the same anatomical location, thus obtaining two different measurements related to blood pressure at one location. The measurements are sufficiently close in time that they may be considered simultaneous. For example the measurements may be interleaved over the time of a measurement cycle.

The sensors comprise ultrasound sensors which may be configured to receive a reflected echo signal as part of an ultrasound imaging process, or they may receive a pressure signal relating to the prevailing pressure or force applied to the sensor.

As the blood flow 110 increases through the peripheral blood vessel 104, the diameter of the blood vessel 104 increases and then decreases, in response to the changes in blood pressure. The changes in diameter force the skin 106 to bulge out. Thus, the blood pressure changes can be monitored from the pressure at the sensor patch 102 on the surface of the skin 106.

Fig. 2 shows a pressure waveform 202 measured at the skin 106 of a subject. Time is shown on the x axis and the pressure on the sensor patch from the skin 106 is shown on the y axis. The pressure waveform 202 is based on measuring the force from the skin 106 of the subject and gives an indication of the blood pressure in the peripheral blood vessel 104.

The waveform comprises the repeating signal shape (i.e. repeating cycles 204) with a period of the heart beat period.

When used for ultrasound imaging, the array of sensors from the sensor patch 102 may be used to capture an image of the vessel beneath the skin surface. Image analysis may then be used to measure the diameter of the peripheral blood vessel 104 by measuring the difference between the two most distal points of the blood vessel 104 crossing a plane 108 extending from the sensor patch 102. The plane 108 may be perpendicular to the skin 106, or may be at other angles to accommodate for peripheral blood vessels 104 which are not parallel to the skin 106. Ideally, the plane 108 is perpendicular to the peripheral blood vessel 104.

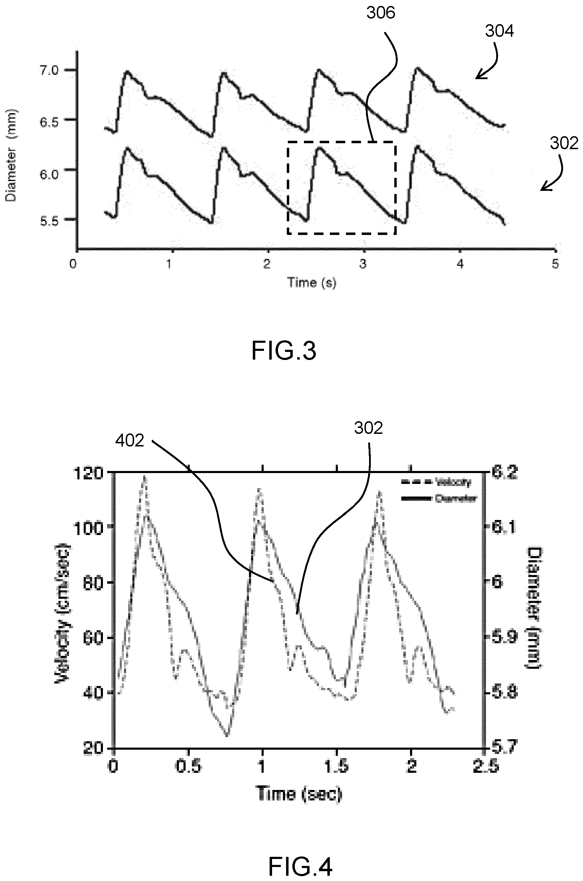

Fig. 3 shows the measured diameter between the opposing inner surfaces and between the opposing outer surfaces of a blood vessel, based on image analysis. The top vessel diameter waveform 304 show the changes in vessel diameter based on outer wall tracking and the bottom vessel diameter waveform 302 shows the changes in vessel diameter based on inner wall tracking. The inner wall refers to the inner wall of a blood vessel and the outer wall refers to the outer wall of the peripheral blood vessel. The vessel diameter waveforms 302, 304 of Fig. 3 can be obtained from M-mode or B-mode ultrasound imaging. One cycle is shown in box 306.

The diameter of the blood vessel increases when the blood pressure inside the blood vessel increases as the walls of the blood vessels are flexible. The vessel diameter waveforms 302,304 may be different for the outer surface and the inner surface as the thickness of the walls of the blood vessel can change when the pressure inside is changed. Typically, the vessel diameter waveform 302 for the inner wall will be used to derive an estimate of the central blood pressure waveform.

Ultrasound imaging is also able to monitor flow conditions. Fig. 4 shows a graph with a velocity waveform 402 (for three cycles) and a corresponding vessel diameter waveform 302. Time is on the x axis, blood velocity is on the right y axis and vessel diameter is on the left y axis. The blood velocity is shown by the dotted line and the vessel diameter is shown by the black uninterrupted line. The blood velocity (in the blood vessel) can be obtained by using Doppler spectrum imaging with the sensor patch 102.

The volume flow of the blood can thus be extracted by multiplying the mean velocity by the area of the blood vessel 104. The area could be obtained by assuming the blood vessel 104 is circular and using the mean diameter from the vessel diameter waveform 302.

A quality factor could be determined for each cycle of the pressure waveform 202 and for each cycle of the vessel diameter waveform 302. Irregular cycles and cycles with a large amount of noise would have a low quality factor. Cycles with a low quality factor may be discarded. The quality factor may be a numerical value (i.e. any number between 0 and 1) or a binary factor (i.e. 0 for low quality and 1 for appropriate quality). The quality factor could, for example, be determined by comparing the minimum, maximum and/or mean variations of the cycles with pre-determined threshold ranges.

In one example, an ensemble waveform is created from the cycles 204 of the pressure waveform 202 and the cycles 306 of the vessel diameter waveform 302. Using the same timing landmark (e.g. the minimum/foot of the cycle as it represents the start of each heartbeat), multiple cycles (with an adequate quality factor) are then combined to create an average waveform. The cycles used may be weighted based on quality factor.

For example, an ensemble pressure waveform may be created from five different cycles in order to filter out trivial differences between each one of the individual cycles of the pressure waveform 202. In another example, the pressure waveform 202 for a subject may be measured for ten seconds for example providing nine pressure waveform cycles. However, the subject may have moved during the 10 seconds and thus one of the pressure waveform cycles may have a different shape to the others. This different pressure waveform cycle would thus have a low quality factor and be discarded before the ensemble pressure waveform is created.

Either one of the ensemble pressure waveform or the ensemble vessel diameter waveform can be used to estimate the central blood pressure.

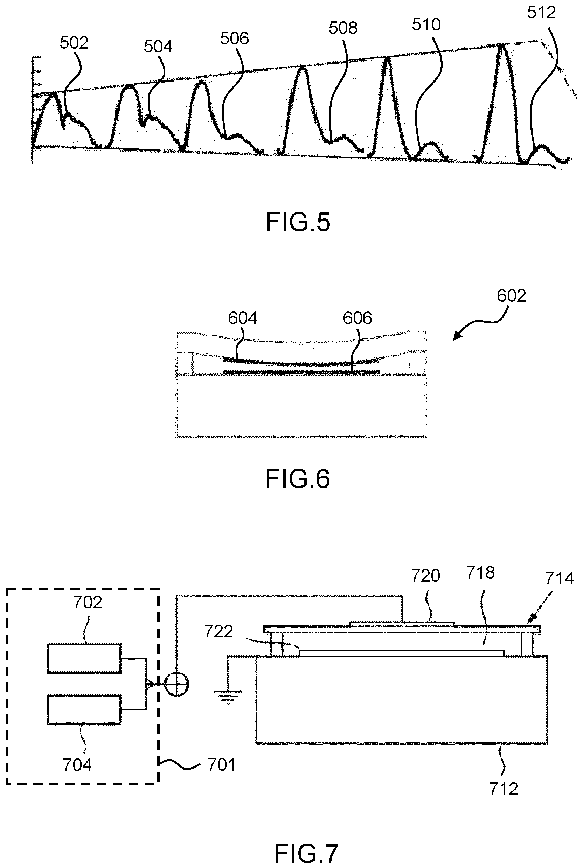

Fig. 5 shows example pressure waveform cycles at different anatomical locations. The first waveform cycle 502 is for the ascending aorta, the second waveform cycle 504 is for the thoracic aorta, the third waveform cycle 506 is for the middle of the abdominal aorta, the fourth waveform cycle 508 is for the distal end of the abdominal aorta, the fifth waveform cycle 510 is for the femoral location and the sixth waveform cycle 512 is for the saphenous location. The central blood pressure may be, for example, for the ascending aorta or the thoracic aorta.

The ensemble pressure waveform for one location can be transformed to a central location by using a transfer function. A frequency-based transfer function can correct for pressure wave distortions caused by bifurcations, changes in vessel diameter and other factors. Alternatively, a physics-based model can be used (viscoelastic tubes used to simulate blood vessels) to determine a transfer function. Thus, the transfer function transforms the ensemble pressure waveform to a central pressure waveform.

A frequency-based approach is for example described in the article "Validation of the transfer function technique for generating central from peripheral upper limb pressure waveform", David Gallagher et. al., American Journal of Hypertension, vol. 17, issue 11, Nov 2004, pp. 1059-1067.

A physics based approach is for example described in the article "Derivation of the ascending aortic-carotid pressure transfer function with an arterial model", M, Karamanoglu et. al., American Journal of Physiology, Heart and Circulatory Physiology, vol. 271, no. 6, 1 December 1996.

The ensemble vessel diameter waveform can be transformed to a second pressure waveform for a peripheral location and said second pressure waveform can then be further transformed to the pressure waveform for a central location (i.e. central pressure waveform).

The morphology of the diameter waveforms is very similar to the pressure waveform, this can be seen in the article "Relationship between the pressure and diameter of the carotid artery in humans.", Sugawara, M., Niki, K., Furuhata, H. et al. Heart Vessels 15, 49-51 (2000).

The diameter waveform is converted to a second pressure waveform by calibrating via cuff pressure measurement. Once calibrated via the cuff pressure measurements, the second pressure waveform can then be transformed from a peripheral blood pressure to central blood pressure via the frequency based transfer function or the physics based approach.

The central pressure waveform can be obtained from either or both of the ensemble pressure waveform and the ensemble vessel diameter waveform. If it is obtained from both, a combination of the central pressure waveforms obtained from each method can be used (e.g. weighted average).

The ensemble pressure waveform and the ensemble vessel diameter waveform could have different artefacts in the waveforms. Thus, the combination of both may not be as useful as one of them individually. Only one of them may be used in these situations for determining the central pressure waveform.

The weighting of each method may, for example, depend on the quality factors of the waveforms used. The central pressure waveform could also be obtained from only the pressure waveform 202 and/or only the vessel diameter waveform 302. However, an ensemble of waveform cycles is not necessary and a single cycle for each of the waveforms could be used.

The central blood pressure waveforms obtained from both methods are typically not calibrated to real blood pressure values. Instead, the methods only provide an accurate waveform shape.

As can be seen in Fig. 5, the further away the pressure is measured from the central blood pressure, the higher the peak of the waveform. However, the mean value of a pressure waveform and the minimum (diastolic) values stay relatively constant irrespective of anatomical location.

Thus, mean values and diastolic values from a pressure cuff (or other apparatus which can accurately measure mean blood pressure and diastolic blood pressure at any anatomical location) can be used to calibrate the central pressure waveform. This is done by ensuring the diastolic value and mean value of both the pressure cuff values and the central pressure waveform are made equal. The central blood pressure can then be determined from the central pressure waveform (i.e. systolic, diastolic and mean blood pressure values as well as general shape of the waveform). The calibrated central pressure waveform is then typically displayed on a monitor.

In a preferred implementation, MEMs devices are used for both the pressure sensing and the ultrasound imaging. Such devices enable on-chip pressure sensing capability, and hence are system components in a variety of applications. MEMS pressure sensors can be classified based on the sensing method used.

A piezoelectric sensor will detect the applied pressure by changing its electrical potential as the output of the sensor. When a pressure is applied, the diaphragm of a piezoelectric pressure sensor is deformed. An electrical voltage is generated due to this deformation. The piezoelectric pressure sensor consists of metallized quartz or ceramic material as sensing element. Normally, this type of pressure sensor is designed together with an amplifier to enhance the electrical interface. An array of piezoelectric sensors can also be used for ultrasound imaging. However, a piezoelectric pressure sensor is very susceptible to shock and vibration.

A piezoresistive pressure sensor instead provides a change in electrical resistance. When a pressure is applied, the diaphragm is flexed as well as the piezoresistive material. This means that the applied pressure causes the piezoresistive material to become slightly longer and thinner. This deformation results in a change in resistance of the piezoresistive material. Thus, by measuring the output voltage of a monitoring circuit, such as a Wheatstone bridge circuit, the change in pressure can be determined and hence the pressure applied to the sensor. An array of piezoresistive sensors can also be used for ultrasound imaging.

Fig. 6 shows an example of a capacitive cMUT sensor 602. This is the preferred sensor implementation. The sensor 602 experiences a change in capacitance proportional to the applied pressure. The sensor 602 has two conductive plates, namely a measuring plate 604 and a reference plate 606. The measuring plate 604 will be flexed when it is subjected to a pressure, whereas the reference plate 606 acts as the reference to the measuring plate 604 with fixed position. Once the measuring plate 604 is flexed, the distance between these two plates is altered. A pressure applied to the measuring plate will change the capacitance and, thus, the capacitive sensor 602 can be used as a pressure sensor.

The capacitive sensor 602 has some advantages over the piezoresistive and piezoelectric pressure sensors. It has very stable operation and the output of the measurement is highly linear.

Fig. 7 shows a known design of cMUT element (a so-called cell) for use in the sensor patch 102. The cMUT element comprises a flexible membrane or diaphragm 714 suspended above a silicon substrate 712 with a gap or cavity 718 there between. A first electrode 722 is located on the floor of the cell on the upper surface of the substrate 712 in this example. A second electrode 720 is located on the diaphragm 714 and moves with the diaphragm. In the example shown, the two electrodes are circular.

A dielectric (not shown) is provided on the substrate 712 and underneath the top (second) electrode 720. The diaphragm may instead function as a dielectric layer.

Preferably, there are two dielectrics which may be equal in composition and thickness, but may be also asymmetric (different materials and thicknesses).

The membrane layer 714 is fixed relative to the top face of the substrate layer 712 and configured and dimensioned so as to define a spherical or cylindrical cavity 718 between the membrane layer 714 and the substrate layer 712.

Other realizations of the electrode 720 design can be considered, such as electrode 720 may be embedded in the membrane 714 or it may be deposited on the membrane 714 as an additional layer.

The first electrode may be directly exposed to the gap 718 or separated from the gap 718 by an electrically insulating layer or film to prevent a short-circuit between the second electrode 720 and the first electrode 722.

In Fig. 7 the first electrode 722 is grounded by way of example. Other arrangements, e.g. a grounded second electrode 720 or both second electrode 720 and first electrode 722 floating are of course equally feasible.

The electrodes of the cMUT element provide the capacitive plates of the device and the gap 718 is the main dielectric between the plates of the capacitor. When the diaphragm vibrates, the changing dimension of the dielectric gap between the plates provides a changing capacitance which is sensed as the response of the cMUT element to a received acoustic echo, when operating in an imaging mode.

The spacing between the electrodes is controlled by applying a static voltage, e.g. a DC bias voltage, to the electrodes with a voltage supply 701. The voltage supply 701 may optionally comprise separate stages 702, 704 for providing the DC and AC or stimulus components respectively of the drive voltage of the cMUT elements, e.g. in transmission mode. The first stage 702 may be adapted to generate the static (DC) voltage component and the second stage 704 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the cMUT element into its collapsed state. This has the advantage that the first stage 702 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

It is known that by applying a static voltage above a certain threshold, the cMUT element is forced into a collapsed state in which the membrane 714 collapses onto the substrate 712. This threshold value may depend on the exact design of the cMUT element and is defined as the DC bias voltage, known as the collapse voltage, at which the membrane 714 sticks to (contacts) the cell floor through the force due to the electric field between the electrodes. The amount (area) of contact between the membrane 714 and the substrate 712 is dependent on the applied bias voltage.

Increasing the contact area between the membrane 714 and the substrate 712 increases the resonant frequency of the membrane 714.

The frequency response of a collapsed mode cMUT element may be varied by adjusting the DC bias voltage applied to the cMUT electrodes after collapse. As a result, the resonant frequency of the cMUT element increases as a higher DC bias voltage is applied to the electrodes.

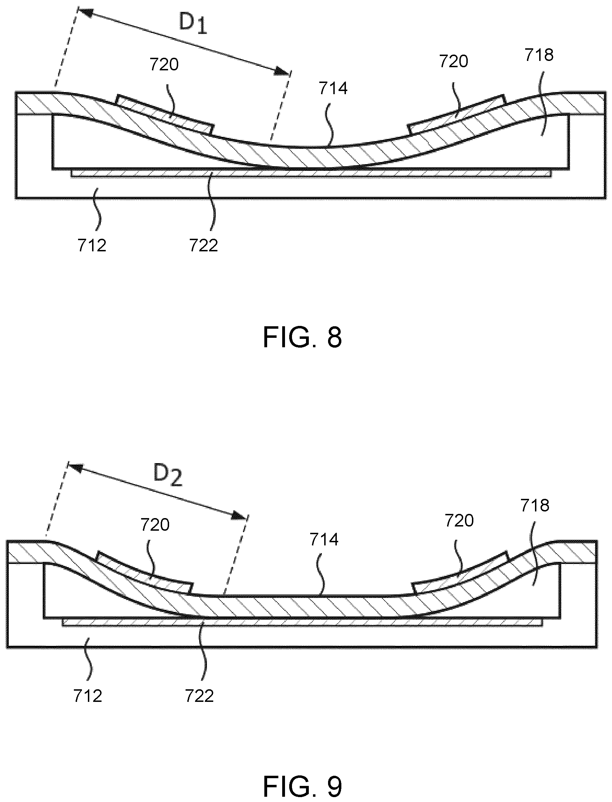

The principles behind this phenomenon are illustrated in Figs. 8 and 9. The cross-sectional views of Figs. 8 and 9 illustrate this one-dimensionally by the distances D1 and D2 between the outer support of the membrane 714 and the point where the membrane begins to touch the floor of the cavity 718 in each illustration. It can be seen that the distance D1 is a relatively long distance in Fig. 8 when a relatively low bias voltage is applied, whereas the distance D2 in Fig. 9 is a much shorter distance due to a higher bias voltage being applied. These distances can be compared to long and short strings which are held by the ends and then plucked. The long, relaxed string will vibrate at a much lower frequency when plucked than will the shorter, tighter string. Analogously, the resonant frequency of the cMUT element in Fig. 8 will be lower than the resonant frequency of the cMUT element in Fig. 9 which is subject to the higher bias voltage.

Thus, a typical cMUT design comprises a flexible membrane or diaphragm, for example formed of silicon nitride, suspended above a silicon substrate with a gap or cavity between them. The gap is caused by removing a sacrificial layer during manufacture. A first electrode is located on the floor of the cell on the upper surface of the substrate and a second electrode is located on the diaphragm and moves with the diaphragm. This same architecture may be used as a pressure sensor or as an ultrasound transmitter and receiver (which involves measuring the pressure of an ultrasound echo wave).

The operation of cMUT elements will be well known to those skilled in the art.

Currently, assessing both the pressure and imaging the blood vessels require two separate devices. The sensor patch of the invention enables these two functions to be combined in a single device, giving benefits in workflow, manufacturing and cost points of view. The sensor patch can thus send and receive ultrasound signals and measure pressure at the same location with a single transducer array.

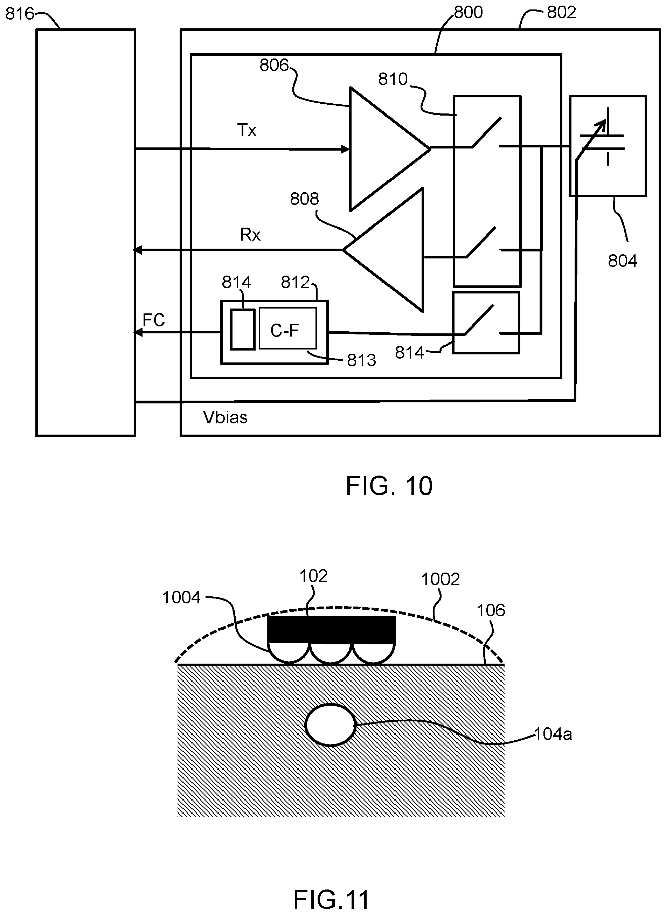

Fig. 10 is used to show how the same sensor array may be used for ultrasound imaging and for pressure sensing. The device comprises a controller 800 provided on an ASIC 802. An array of cMUT devices 804 is provided, for example monolithically integrated with the ASIC 802. Each cMUT device may comprise a number of actual cMUT elements (i.e. cells).

The controller 800 comprises an ultrasound transmit circuit 806 and an ultrasound receive circuit 808 for each cMUT device 804, for an imaging mode. A switch arrangement 810 for each cMUT device couples the receive and transmit circuits to the cMUT device 804. A system controller 816 controls the timing of when to transmit and when to start receiving and hence controls the switch arrangements 810 for the different cMUT devices. The cMUT devices are connected to the transmit and receive circuits for an imaging mode, or else all cMUT devices are isolated from the transmit and receive circuits for a separate pressure sensing mode, also controlled by the system controller 816.

The controller further comprises a measuring circuit 812 for measuring the capacitance (or more generally any a pressure-dependent electrical characteristic) of the cMUT device. The measuring circuit 812 connects to the cMUT device through a second switch 814. There is again a second switch 814 for each cMUT device 804. However, a single measuring circuit 812 may be provided. Thus, the cMUT devices 804 may all be connected electrically in parallel for the pressure sensing mode. If there are multiple ASIC dies each with a respective sub-array of the cMUT devices, there may then be a measuring circuit per semiconductor die.

To measure the capacitance, the measuring circuit 812 comprises an oscillator circuit 813 which combines with the cMUT device to define the oscillation characteristics, and an output circuit 814 for providing an output signal FC which depends on the oscillator frequency.

The measuring circuit 812 in this example comprises an oscillator 813 to measure the cMUT capacitance, which depends on the pressure being sensed. The oscillator is thus one example of a capacitance-to-frequency converter circuit.

The ASIC 802 connects to the system controller 816, which provides the control commands for transmission, processes the received reflected echo signals, and receives the pressure measurement signal (output signal FC in this example). The module 816 also provides the bias voltage to the cMUT devices. The bias voltage is for example the same for all cMUT devices of the array.

During the ultrasound mode imaging mode, the system controller 816 sends the request for a transmit event to the ASIC, and the ASIC then delivers the high voltage pulses to the cMUT devices. The ASIC receive the acoustic echo signal and sends it to the system controller. The switches of the switch arrangement 810 are closed and the switch 814 is open.

During the pressure sensor mode, the switches of the switch arrangement 810 are open, and the switch 814 is closed. The measuring circuit 812 of the ASIC converts the capacitance value of the cMUT into the output signal signal FC. The frequency of the signal FC and the capacitance of the cMUT are related.

When the pressure changes, the capacitance of the cMUT changes and hence the resonant oscillation frequency represented by the output signal FC changes accordingly.

The example of Fig. 10 is based on converting the capacitance of the cMUT device (or more accurately the parallel combination of the cMUT devices of the ASIC) into a frequency. The capacitance may instead be converted into a signal having a duty cycle, voltage or current which represents the capacitance. The cMUT capacitance may instead be converted into a digital signal.

An ultrasound image is formed of frames, where each frame consists of multiple fire and receive events. The pressure sensing can take place after each frame or after sequences of multiple frames. In this way, the image generation and the pressure sensing are performed in real time. Each imaging frame for example comprises a transmit period and a receive period. Typically, the frame duration is around 20µs and the pressure sensing function has a minimum duration of around 10µs.

This provides a sequential pressure sensing mode and imaging mode. The pressure may be sensed when the ultrasound image is being reconstructed. The pressure sensing and imaging are this effectively determined in the same time frame.

The pressure sensing does not need to be updated at such a fast rate (every 30µs in this example), so it is possible to reduce the time impact of the pressure sensing on the frame rate by reducing the rate at which pressure measurements are taken. Pressure sensing periods may thus be performed every N imaging frames. Each imaging frame again comprises a transmit period and a receive period.

The value N can be of the order of 1000. With a resulting combined frame duration of 20ms, the pressure reading duration is negligible in comparison, but the pressure is updated at a sufficiently high rate (50Hz in this example). Even less frequent pressure measurement is of course possible.

There are other ways to use a cMUT cell as a pressure sensor. For example, a closed loop feedback path may be used to maintain the capacitance of the cMUT device constant by adjusting the bias voltage Vbias. An oscillator circuit may again be used for converting capacitance to frequency. The output frequency may then be compared with a fixed reference frequency and the error signal may be used in a phase locked loop (PLL) control system which tunes the bias voltage Vbias of the cMUT device, such that the output frequency is constant and equal to the reference frequency.

In another example, a resonance frequency of the cMUT device may be measured (rather than an oscillation frequency of an oscillator circuit which includes the cMUT device capacitance). Methods suitable for measuring a resonance frequency of a cMUT are well known in the art.

The flexible diaphragm of the sensors (whether piezoelectric, piezoresistive or capacitive) are mounted along the periphery of a blood vessel 104. MEMs devices of this type are very sensitive to the blood pressure. Pressure exerted anywhere in a confined incompressible fluid is transmitted equally in all directions throughout the fluid such that the pressure variations remain the same. As such, equal force is transmitted along the surface of the diaphragm of the sensors. The pressure corresonding to no deflection or a reference deflection is known and can be used to calibrate the measurements.

The same sensors (whether piezoelectric, piezoresistive or capacitive) are used for both pressure sensing capabilities and ultrasound imaging capabilities. The integrated ultrasound transducer capability is used to determine the vessel diameter. The measurement can be done using M-mode or B-mode imaging. The diameter can be found using an automatic algorithm to track the vessel using color Doppler mode and extract the diameter real time. The beat-to-beat volume flow can be extracted from the velocity waveform 402 using spectral Doppler and the measured diameter of the vessel.

Both the pressure waveform 202 and the vessel diameter waveform 302 can be used to estimate a central aortic waveform. By combining both approaches (force-based and vessel diameter), a robust central pressure estimation can be achieved more consistently.

Fig. 11 shows a wearable device with a sensing patch 102. The use of capacitive sensors also opens the option for wearable concepts. As the ultrasound imaging capability and pressure sensing capability can be integrated in a single package, the sensor patch 102 can be applied to the body as a wearable concept using an acoustic skin interface 1004. In this example, the sensor patch 102 is attached to a strap 1002 and has an acoustic interface 1004 between the sensor patch 102 and the intended area of contact with the skin 106.

The wearable device could also have a housing element which houses the sensor patch 102 and the required processor. The housing element then has an aperture for the sensor patch 102, such that the sensing area (the part that does the pressure sensing and ultrasound imaging) of the sensing patch 102 would fit through the aperture. The acoustic skin interface 1004 is placed at the aperture. The strap 1002 is designed to fit around a subject's wrist such that the sensor patch 102 can measure the blood pressure from the radial artery 104a.

Alternatively, the sensor patch 102 can be used on the carotid artery, as this blood vessel 104 is relatively closer to the heart than other blood vessels in the periphery of the body (i.e. in the arm/leg).

In summary, an example of the invention involves the following workflow:
(i) Obtain a force signal, and thereby determine surface displacement waveform;
(ii) Obtain an M-mode image, and determine the vessel diameter waveform;
(iii) Determine the quality of the waveform (e.g. periodicity, heart rate etc.), because a frequency-based transfer function should be applied only to periodic waveforms, so irregular waveforms should be discarded;
(iv) Create an ensemble (average) signal of external pressure waveform cycles using a landmark, e.g. from an ECG trace;
(v) Transform to a central aortic pressure waveform using a transfer function; and
(vi) Using cuff pressure values, calibrate the transformed waveform.

The skilled person would be readily capable of developing a processor for carrying out any herein described method. Thus, each step of a flow chart may represent a different action performed by a processor, and may be performed by a respective module of the processing processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A device for estimating the central blood pressure of a subject, the device comprising:
a sensor patch (102) comprising an array of sensors configured to:
measure the an indication of blood pressure of a peripheral blood vessel (104); and
perform ultrasound imaging on the peripheral blood vessel (104), wherein the same sensors are used for blood pressure measurement and ultrasound imaging; and
a processor configured to:
obtain a peripheral pressure signal comprising a pressure waveform (202) from the indication of the blood pressure from the sensor patch (102);
derive an image of the peripheral blood vessel from the signals received from the sensor patch (102);
determine a vessel diameter from the image of the peripheral blood vessel over time as a vessel diameter waveform (302);
derive an estimate of the central blood pressure waveform of the subject from the pressure waveform (202) and/or the vessel diameter waveform (302).

2. The device of claim 1, wherein the processor is configured to:
determine the vessel diameter by applying an automatic tracking algorithm to the image of the peripheral blood vessel in color Doppler mode.

3. The device of any one of claims 1 or 2, wherein the array of sensors comprises:
capacitive micro-machined ultrasound transducers (cMUTs);
piezoelectric sensors; or
piezoresistive sensors.

4. The device of any one of claims 1 to 3, wherein the processor is further adapted to:
determine a quality factor for each of a plurality of peripheral pressure waveform cycles (204) of the pressure waveform and vessel diameter waveform cycles (306) of the vessel diameter waveform;
create an ensemble pressure waveform based on the pressure waveform cycles (204) and their respective quality factors; and
create an ensemble diameter waveform based on the vessel diameter waveform cycles (306) and their respective quality factors,
wherein estimating the central blood pressure is based on the ensemble pressure waveform and/or the ensemble diameter waveform.

5. The device of claim 4, wherein the processor is adapted to derive an estimate of the central blood pressure by:
applying a frequency-based transfer function to the ensemble pressure waveform and/or the ensemble diameter waveform; or
using a physics-based model to transform the ensemble pressure waveform and/or the ensemble diameter waveform to an estimate of the central blood pressure.

6. The device of any one of claims 1 to 5, wherein the processor is further adapted to:
calibrate a mean value and a diastolic value of the estimated central blood pressure to a cuff mean value and a cuff diastolic value obtained from pressure cuff measurements.

7. The device of any one of claims 1 to 6, wherein the processor is further configured to transform the vessel diameter waveform (302) to a second pressure waveform for the peripheral blood vessel and wherein the processor is configured to derive the estimate of the central blood pressure waveform of the subject from the pressure waveform (202) and/or the second pressure waveform.

8. The device of any one of claims 1 to 7, further comprising:
a housing element for housing the sensor patch (102) and the processor, wherein the housing element comprises an opening and wherein the opening is for a sensing area of the sensor patch (102);
a strap (1002) attached the housing element; and
a skin interface (1004) at the opening of the housing element.

9. A method for estimating the central blood pressure of a subject, the method comprising:
measuring an indication of blood pressure of a peripheral blood vessel (104) to derive a pressure waveform (202) from sensors of a sensor patch (102);
performing ultrasound imaging on the peripheral blood vessel (104) using the same sensors as are used for the blood pressure measurement;
determining a vessel diameter from the image of the peripheral blood vessel (104) over time as a vessel diameter waveform (302); and
deriving an estimate of the central blood pressure waveform of the subject from the pressure waveform (202) and/or the vessel diameter waveform (302).

10. The method of claim 9, wherein determining the vessel diameter is determined by applying an automatic tracking algorithm to the image in color Doppler mode.

11. The method of any one of claims 9 or 10, further comprising:
determining a quality factor for a plurality of peripheral pressure waveform cycles (204) of the pressure waveform and a plurality of vessel diameter waveform cycles (306) of the vessel diameter waveform;
creating an ensemble pressure waveform based on the pressure waveform cycles (204) and their respective quality factors; and
creating an ensemble diameter waveform based on the vessel diameter waveform cycles (306) and their respective quality factors,
wherein estimating the central blood pressure is based on the ensemble pressure waveform and/or the ensemble diameter waveform.

12. The method of claim 11, wherein estimating the central blood pressure is based on:
applying a frequency-based transfer function to the ensemble pressure waveform and/or the ensemble diameter waveform; or
using a physics-based model to transform the ensemble pressure waveform and/or the ensemble diameter waveform to an estimate of the central blood pressure.

13. The method of any one of claims 9 to 12, further comprising calibrating a mean value and a diastolic value of the estimated central blood pressure to a cuff mean value and a cuff diastolic value obtained from pressure cuff measurements.

14. The method of any one of claims 9 to 13, further comprising transforming the vessel diameter waveform (302) to a second pressure waveform for the peripheral blood vessel and deriving the estimate of the central blood pressure waveform of the subject from the pressure waveform (202) and/or the second pressure waveform.

15. A computer program comprising computer program code which, when executed by a processor, causes the processor to perform the method of any one of claims 9 to 14.
